Europäisches Patentamt
European Patent Office
Office européen des brevets

(19) (11) Publication number: **0 310 931 A2**

# (12) EUROPEAN PATENT APPLICATION

(21) Application number: 88116013.9

(51) Int. Cl.[4]: A23K 1/16 , A23K 1/18

(22) Date of filing: 28.09.88

(30) Priority: 08.10.87 US 105902

(43) Date of publication of application: 12.04.89 Bulletin 89/15

(84) Designated Contracting States: AT BE CH DE ES FR GB GR IT LI LU NL SE

(71) Applicant: ETHYL CORPORATION
Ethyl Tower 451 Florida Boulevard
Baton Rouge Louisiana 70801(US)

(72) Inventor: Laurent, Sebastian Marc
Route 6, Box 492
Greenwell Spings Louisiana 70739(US)
Inventor: Ramachandran, Venkataraman
1446 Brookhollow Drive
Baton Rouge Louisiana 70810(US)

(74) Representative: Sandmair, Kurt, Dr. Dr. et al
Patentanw lte Schwabe, Sandmair, Marx
Postfach 86 02 45 Stuntzstrasse 16
D-8000 München 86(DE)

## (54) Poultry feed.

(57) A compound corresponding to the formula:

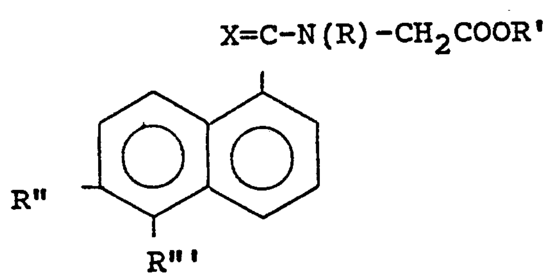

wherein R is an alkyl group of 1-6 carbons, R′ is hydrogen or a saturated hydrocarbyl group, R″ is hydrogen, hydroxy, or an alkyl or alkoxy group of 1-6 carbons, R‴ is hydro gen or $(CF_2)_nCF_3$, X is sulfur or oxygen, and n is zero or an integer of at least one, or a therapeutically acceptable salt thereof is added to a poultry feed as an antilipogenic agent to provide a novel composition which decreases lipid levels and increases lean/fat ratios in poultry fed therewith. The preferred antilipogenic agent is tolrestat.

EP 0 310 931 A2

## POULTRY FEED

This invention relates to poultry feed and more particularly to a feed that is effective in lowering lipid levels in poultry.

As disclosed in U.S. patent 4,614,746 (Asato et al.), it is known that antilipogenic agents are useful for increasing the lean/fat ratio in poultry and other warm-blooded animals. U.S. Patent 4,492,706 (Kallai-Sanfacon) teaches that certain aldose reductase inhibitors, such as N-[(6-methoxy-5-trifluoromethyl-1-naphthalenyl)thiono]-N-methylglycine (commonly known as tolrestat), function as antilipogenic agents in mammals. U.S. Patents 4,439,617 (Sestanj et al.), 4,604,406 (Bellini et al.), and 4,699,993 (Brandt) disclose the utility of tolrestat and related compounds as aldose reductase inhibitors.

An object of this invention is to provide a means of lowering lipid levels in poultry.

Another object is to provide a means of increasing the lean/fat ratio in poultry.

These and other objects are attained by the provision of a poultry feed containing an effective amount of an antilipogenic agent selected from (1) a compound corresponding to the formula:

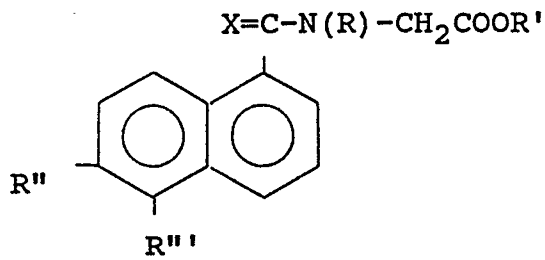

wherein R is an alkyl group of 1-6 carbons, R′ is hydrogen or a saturated hydrocarbyl group, R″ is hydrogen, hydroxy, or an alkyl or alkoxy group of 1-6 carbons, R‴ is hydrogen or $(CF_2)_nCF_3$, X is sulfur or oxygen, and n is zero or an integer of at least one and (2) a therapeutically acceptable salt of the compound with an organic or inorganic base.

The poultry feed to which the antilipogenic agent is added may be any of the feeds commonly employed for poultry, i.e., a feed comprising conventional poultry feed nutrients and additives, such as sources of carbohydrate, protein, fat, fiber, vitamins, minerals, and disease inhibitors. As is known, such feeds generally include at least one of wheat, barley, soybean, corn meal and/or bone materials, flax, fishmeal, peanut meal, sugar beet pulp, cottonseed hull and meal, sugar cane pulp, sorghum, rye, rice, and the like; and they usually contain at least 10% by weight of crude protein and at least 1% by weight of crude fat. For example, a feed may contain 50-75% corn, 15-35% soybean meal, 0.5-9% limestone, 0.6-2% phosphates, 1-7% fat, and 0.1-1% vitamins, amino acids, salt, and other minerals, with other ingredients, such as additional fiber, antibiotics, and preservatives, also being included when desired.

The antilipogenic agent which is included in the poultry feed may be any compound corresponding to the above formula or a therapeutically acceptable salt thereof, but it is preferably a compound wherein R of the formula is an alkyl group of 1-6 carbons, R′ is hydrogen, R″ is an alkoxy group of 1-6 carbons, R‴ is $(CF_2)_nCF_3$, X is sulfur, and n is zero or an integer of 1-5. Such compounds are disclosed in Sestanj et al., Bellini et al., and Brandt. Exemplary of the antilipogenic agents are tolrestat, sodium tolrestat, tetramethylammonium tolrestat, methyl tolrestat, phenyl tolrestat, N-[(6-methoxy-5-trifluoro-methyl-1-naphthalenyl)thiono]-N-ethylglycine, N-[(6-methyl-5-trifluoromethyl-1-naphthalenyl)thiono]-N-methylglycine, N-[(6-methoxy-5-pentafluoroethyl-1-naphthalenyl)thiono]-N-methylglycine, N-(6-methoxy-5-heptafluoropropyl-l naphthoyl)-N-methylglycine, N-[(6-hydroxy-5-pentafluoroethyl-1-naphthalenyl)thiono]-N-methylglycine, N-[(5-pentafluoroethyl-1- naphthalenyl)thiono] N-methylglycine, and N-[(6-methoxy-1 naphthalenyl)thiono]-N-methylglycine. The preferred antilipogenic agent is tolrestat.

The amount of antilipogenic agent incorporated into the poultry feed should be an effective amount, i.e., an amount that has an antilipogenic effect. This may vary with factors such as the particular heredity of the poultry to be fed, since some birds are bred to be naturally leaner than others. However, the amount of antilipogenic agent in the feed is generally at least about 1.0 ppm. Most commonly the amount is in the range of 1-1000 ppm, preferably 1-100 ppm, and most preferably 5-50 ppm.

The invention is advantageous in that chickens, turkeys, geese, ducks, and other poultry which are fed the novel compositions have lower lipid levels and a higher lean/fat ratio than they would have had if they

had been fed a conventional poultry feed. Moreover, this advantage is achieved without any significant effect on total body weight, body weight gain, feed consumption, or feed conversion.

The following example is given to illustrate the invention and is not intended as a limitation thereof.

## EXAMPLE

Three poultry feeds were prepared by blending 16 pounds of a corn concentrate with 784 pounds of a basal composition comprising about 54% corn, 34% soybean meal, 2.5% calcium diphosphate, 0.25% oyster shell flour, 0.25% mineral mix, 0.25% vitamin mix, 0.12% DL-methionine, and 7% tallow. The concentrate used to prepare a control feed contained only corn. The concentrate used to prepare Feed A consisted of 15.84 pounds of corn and 0.16 pound of tolrestat. The concentrate used to prepare Feed B consisted of 15.68 pounds of corn and 0.32 pound of tolrestat.

Ten female broilers per pen were housed at one week of age and placed on Control Feed, Feed A, or Feed B diets to provide them with a 0, 20, or 40 ppm daily intake of tolrestat for six weeks. Twenty-four total pens with eight replications were used (240 total birds). Twelve missexed birds were removed as soon as they could be identified, and all data were corrected for these birds. Body weight was determined at l, 4, and 7 weeks of age, feed consumption data were collected from ages 1-4 weeks and 4-7 weeks, blood plasma absorbance was determined on a maximum of eight females per pen at 7 weeks of age, and the amount of fat in those birds was calculated from the blood plasma absorbance data as in Griffin et al., British Poultry Science, Vol. 23, pp. 307-313, 1982. The test results are shown in Tables 1-4.

TABLE 1

Effect of tolrestat on body weight (g) of female broilers.

| Tolrestat (ppm) | Weeks of Age | | |
|---|---|---|---|
| | 1 | 4 | 7 |
| 0 | 124 ± 03 | 881 ± 10 | 2051 ± 16 |
| 20 | 127 ± 02 | 895 ± 12 | 2043 ± 21 |
| 40 | 126 ± 01 | 909 ± 13 | 2081 ± 25 |

TABLE 2

Effect of tolrestat on body weight gain (kg) of female broilers.

| Tolrestat (ppm) | Weeks of Age | |
|---|---|---|
| | 1-4 | 4-7 |
| 0 | 0.757 ± .008 | 1.169 ± .011 |
| 20 | 0.768 ± .011 | 1.144 ± .014 |
| 40 | 0.783 ± .012 | 1.172 ± .018 |

TABLE 3

| Effect of tolrestat on feed consumption (kg) and feed conversion (kg feed/kg body weight gain) of female broilers. | | | | |
|---|---|---|---|---|
| Tolrestat (ppm) | Weeks of Age | | | |
| | 1-4 | | 4-7 | |
| | Consumption | Conversion | Consumption | Conversion |
| 0 | 1.311 ± .028 | 1.71 ± .03 | 2.643 ± 53 | 2.22 ± .05 |
| 20 | 1.303 ± .018 | 1.72 ± .02 | 2.556 ± 38 | 2.28 ± .06 |
| 40 | 1.359 ± .028 | 1.74 ± .02 | 2.658 ± 39 | 2.27 ± .04 |

TABLE 4

| Effect of tolrestat on estimated body fat (g fat/kg fresh weight). | |
|---|---|
| Tolrestat (ppm) | Body Fat (g/kg fresh weight) |
| 0 | 227 |
| 20 | 194 |
| 40 | 161 |

## Claims

1. A poultry feed containing an effective amount of an antilipogenic agent selected from (1) a compound corresponding to the formula:

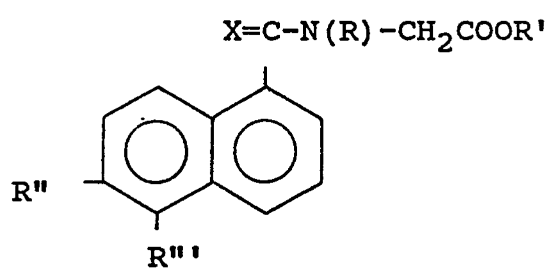

wherein R is an alkyl group of 1-6 carbons, R′ is hydrogen or a saturated hydrocarbyl group, R″ is hydrogen, hydroxy, or an alkyl or alkoxy group of 1-6 carbons, R‴ is hydrogen or $(CF_2)_nCF_3$, X is sulfur or oxygen, and n is zero or an integer of at least one and (2) a therapeutically acceptable salt of the compound with an organic or inorganic base.

2. The poultry feed of claim 1 comprising a mixture of conventional poultry feed nutrients and additives and at least about 1.0 ppm of the antilipogenic agent.

3. The poultry feed of claim 2 containing 1-1000 ppm of the antilipogenic agent.

4. The poultry feed of claim 3 containing 1-100 ppm of the antilipogenic agent.

5. The poultry feed of claim 4 containing 5-50 ppm of the antilipogenic agent.

6. The poultry feed of claim 1 wherein the antilipogenic agent is a compound corresponding to the formula:

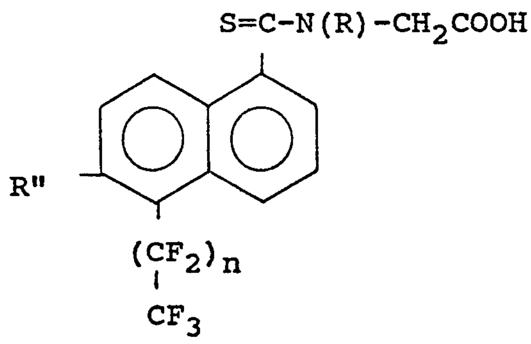

in which R is an alkyl group of 1-6 carbons, R″ is an alkoxy group of 1-6 carbons, and n is zero or an integer of 1-5.

7. The poultry feed of claim 6 wherein the antilipogenic agent is tolrestat.

8. The poultry feed of claim I comprising a mixture of conventional poultry feed nutrients and additives and 5-50 ppm of tolrestat as the antilipogenic agent.

9. A process for lowering lipid levels in poultry which comprises feeding poultry a poultry feed containing an effective amount of an antilipogenic agent selected from (1) a compound corresponding to the formula:

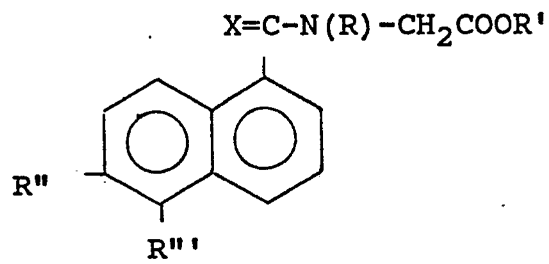

wherein R is an alkyl group of 1-6 carbons, R′ is hydrogen or a saturated hydrocarbyl group, R″ is hydrogen, hydroxy, or an alkyl or alkoxy group of 1-6 carbons, R‴ is hydrogen or $(CF_2)_nCF_3$, X is sulfur or oxygen, and n is zero or an integer of at least one and (2) a therapeutically acceptable salt of the compound with an organic or inorganic base.

10. A process for increasing the lean/fat ratio in poultry which comprises feeding poultry a poultry feed containing an effective amount of an antilipogenic agent selected from (1) a compound corresponding to the formula:

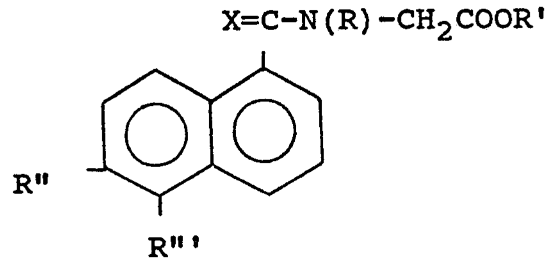

wherein R is an alkyl group of 1-6 carbons, R′ is hydrogen or a saturated hydrocarbyl group, R″ is hydrogen, hydroxy, or an alkyl or alkoxy group of 1-6 carbons, R‴ is hydrogen or $(CF_2)_nCF_3$, X is sulfur or oxygen, and n is zero or an integer of at least one and (2) a therapeutically acceptable salt of the compound with an organic or inorganic base.